**Europäisches Patentamt**

**European Patent Office**

**Office européen des brevets**

(19)

(11) Numéro de publication : **0 337 842 B1**

(12)

# FASCICULE DE BREVET EUROPEEN

(45) Date de publication du fascicule du brevet :
**10.06.92 Bulletin 92/24**

(51) Int. Cl.⁵ : **G01N 3/00, A61B 5/103**

(21) Numéro de dépôt : **89400866.3**

(22) Date de dépôt : **29.03.89**

(54) **Procédé et dispositif pour mesurer l'élasticité d'une couche superficielle, en particulier de la peau.**

(30) Priorité : **31.03.88 FR 8804292**

(43) Date de publication de la demande :
**18.10.89 Bulletin 89/42**

(45) Mention de la délivrance du brevet :
**10.06.92 Bulletin 92/24**

(84) Etats contractants désignés :
**AT BE CH DE ES GB IT LI NL SE**

(56) Documents cités :
**DE-A- 3 445 587**
**FR-A- 733 686**
**GB-A- 2 068 567**
**ENGINEERING IN MEDICINE, vol. 8, no. 2, avril 1979, pp 105, 106; B.H. SHAW: "Continuous tissue pressure monitor".**

(73) Titulaire : **L'OREAL**
**14, Rue Royale**
**F-75008 Paris (FR)**

(72) Inventeur : **Bazin, Roland**
**45, rue Guy Mocquet**
**F-94400 Vitry S/Seine (FR)**
Inventeur : **Obadia, Gérard**
**1, Villa du Cadra Solaire**
**F-92120 Montrouge (FR)**
Inventeur : **Marcotte, Louis**
**28, rue du Général Leclerc**
**F-94150 Chevilly La Rue - Rungis (FR)**
Inventeur : **Scot, Jean**
**38-40, rue Stephenson**
**F-75018 Paris (FR)**

(74) Mandataire : **Michardière, Bernard et al**
**C/O CABINET PEUSCET 68, rue d'Hauteville**
**F-75010 Paris (FR)**

## Description

L'invention est relative à un procédé pour mesurer l'élasticité d'une couche superficielle, en particulier de la peau, suivant lequel on délimite une zone de cette couche, puis on crée, sur cette zone, une dépression pour provoquer une déformation de ladite zone, et on mesure la dépression nécessaire pour créer une déformation d'amplitude prédéterminée de ladite couche, ladite déformation étant détectée par obturation d'un orifice situé à une distance initiale correspondant à l'amplitude prédéterminée de déformation de la couche superficielle.

Les procédés proposés jusqu'à ce jour, notamment dans le document FR-A-733 686, tout en donnant satisfaction, sont d'une mise en oeuvre relativement délicate et ne permettent pas de réaliser une mesure quasiment automatique et instantanée de l'élasticité.

L'invention a pour but, surtout, de rendre le procédé du genre défini précédemment tel qu'il réponde mieux que jusqu'à présent aux diverses exigences de la pratique et notamment tel, qu'en particulier, il permette une mesure rapide et pratiquement automatique, avec une mise en oeuvre simple.

Selon l'invention, le procédé pour mesurer l'élasticité d'une couche superficielle, en particulier de la peau, du genre défini précédemment, est caractérisé par le fait que ledit orifice est un orifice d'aspiration à travers lequel est crée la dépression et que la dépression mesurée est la dépression, qui existe dans un volume délimité par une gorge annulaire et ladite zone de la couche superficielle.

Avantageusement, la déformation d'amplitude prédéterminée est détectée par obturation d'un orifice d'aspiration situé à une distance initiale prédéterminée de la couche superficielle. Cette distance prédéterminée peut être choisie égale à 2,5 mm.

L'invention est également relative à un dispositif pour mesurer l'élasticité d'une couche superficielle, en particulier de la peau, pour la mise en oeuvre du procédé ci-dessus caractérisé, comprenant une sonde munie d'une cavité délimitée par un rebord propre à être appliqué sur la couche à étudier pour définir la susdite zone, des moyens pour créer une dépression dans la cavité et provoquer la déformation de la zone, un orifice prévu dans la susdite cavité, à une distance initiale correspondant à une amplitude prédéterminée de déformation de la couche superficielle, cet orifice étant disposé de manière à être obturé par la susdite couche lorsque la déformation atteint l'amplitude prédéterminée, et des moyens de mesure de la dépression qui a produit cette déformation d'amplitude prédéterminée, ledit orifice étant un orifice d'aspiration, caractérisé par le fait que la dépression mesurée est la dépression qui existe dans une gorge annulaire dont est munie ladite cavité et qui est en retrait par rapport audit orifice.

Avantageusement, la cavité comporte, dans son fond, une partie en saillie traversée par un passage de liaison avec un système de conduit interne relié à une source de dépression, l'extrémité du passage débouchant dans la cavité constituant le susdit orifice destiné à être obturé par la zone déformée de la couche superficielle.

Avantageusement, la sonde présente une face d'extrémité, destinée à être appliquée contre la couche superficielle, qui se trouve dans un plan situé à une distance de l'ordre de 2,5 mm par rapport au plan du susdit orifice. Le diamètre de la cavité peut être de l'ordre de 15 mm.

La zone centrale de la face d'appui de la sonde contre la couche superficielle est occupée par la susdite cavité, et cette face d'appui comporte, autour de la cavité, des moyens propres à réaliser une étanchéité entre la sonde et la couche superficielle.

Ces moyens pour réaliser une étanchéité peuvent comprendre au moins une gorge périphérique, notamment à section transversale en V renversé, entourant la cavité, cette gorge étant reliée à la source de dépression. De préférence, deux gorges périphériques concentriques sont prévues.

On peut prévoir un clapet propre à s'ouvrir lorsque l'orifice d'aspiration, prévu dans la cavité, est fermé par la couche superficielle déformée.

Selon une réalisation avantageuse, la sonde comporte une chambre fermée, d'un volume supérieur à celui de la cavité, ladite chambre étant reliée par un canal à ladite cavité, l'ensemble étant tel que lorsque l'orifice d'aspiration de la cavité est fermé, la pression dans la chambre reste pratiquement constante. Le dispositif comporte, de préférence, une tubulure débouchant dans ladite chambre et reliée à un capteur de pression pour permettre de mesurer la pression dans la chambre et la cavité, lorsque cette dernière est fermée par la couche superficielle déformée.

La sonde peut avoir une forme cylindrique de révolution, dont la face transversale inférieure est destinée à être appliquée contre la couche superficielle, la partie centrale de cette face inférieure comportant la susdite cavité entourée par un rebord circulaire muni de deux gorges périphériques reliées à la source de dépression, la susdite chambre étant prévue dans une zone intermédiaire, suivant la longueur du cylindre, cette chambre étant reliée à la cavité par un canal dans une région située à l'extérieur de la partie en saillie du fond de la cavité, ladite chambre étant en outre reliée, du côté opposé à la cavité, à une tubulure, coaxiale à une tubulure principale, de plus grand diamètre, reliée à la source de dépression et communiquant à l'intérieur du cylindre avec un système de conduit interne relié respectivement aux gorges périphériques et à l'orifice débouchant dans la cavité.

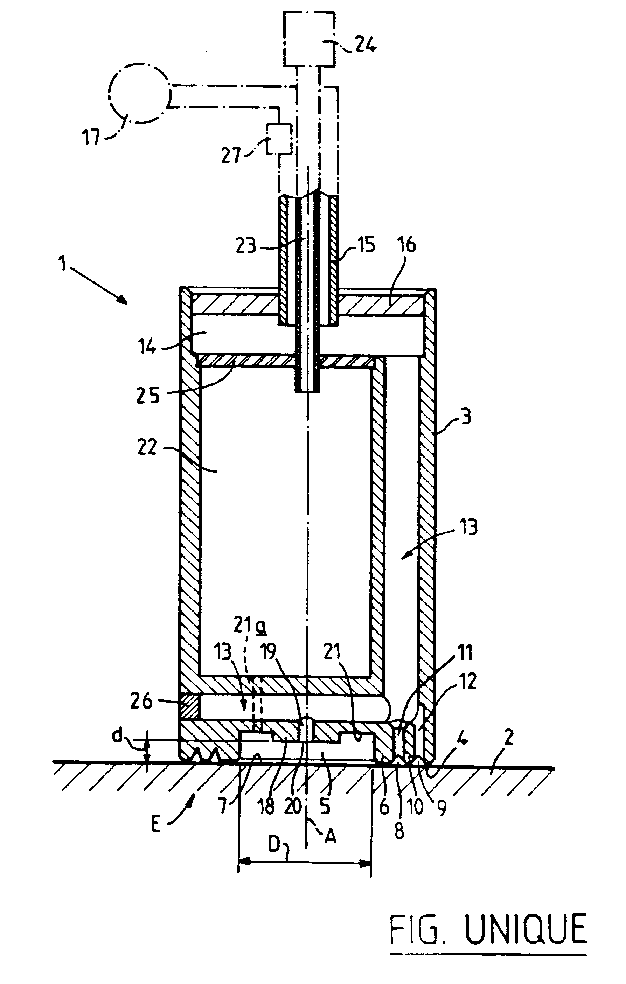

L'invention consiste, mises à part les dispositions exposées ci-dessus, en un certain nombre d'autres dispositions dont il sera plus explicitement question ci-après à propos d'un mode de réalisation particulier décrit avec référence au dessin ci-annexé.

La figure unique de ce dessin est une coupe longitudinale schématique d'un dispositif conforme à l'invention pour mesurer l'élasticité d'une couche superficielle, en particulier de la peau humaine.

En se reportant au dessin, on peut voir un dispositif 1 pour mesurer l'élasticité d'une couche superficielle qui, dans l'exemple de réalisation considéré, est constituée par de la peau 2. Le dispositif 1 comprend une sonde 3, avantageusement de forme cylindrique, qui comporte une face d'appui 4 contre la peau 2.

La face 4 est située dans un plan orthogonal à l'axe A de la sonde. La région centrale de la face 4 est occupée par une cavité 5, avantageusement circulaire, délimitée par un rebord 6 propre à être appliqué sur la peau pour délimiter une zone 7 qui sera soumise à déformation.

La face d'appui 4 comporte, autour de la cavité 5 et du rebord 6, des moyens E propres à réaliser une étanchéité entre la sonde 3 et la peau 2. Ces moyens d'étanchéité E comprennent, avantageusement, deux gorges concentriques 8, 9 centrées sur l'axe A et séparées l'une de l'autre par une nervure 10 propre à s'appliquer sur la peau 2. La section transversale des gorges 8, 9 a une forme en V renversé. Chaque gorge 8, 9 est reliée par un canal respectivement 11, 12 à un système de conduit interne 13 de la sonde qui communique avec un logement 14 prévu vers l'extrémité de la sonde éloignée de la face 4. Une tubulure 15, coaxiale à la sonde 3, traverse la paroi transversale supérieure 16 de la sonde pour déboucher dans le logement 14. Cette tubulure 15, de préférence souple, est branchée à son autre extrémité sur une petite pompe à air 17 à débit constant.

La cavité 5 comporte, dans son fond, une partie en saillie 18 traversée par un passage 19 de liaison avec le système de conduit interne 13 lui-même relié à la source de dépression constituée par la pompe 17. Le diamètre D de la cavité 5 peut être de l'ordre de 15 mm.

L'extrémité du passage 19 débouchant dans la cavité 5 constitue un orifice 2Ø destiné à être obturé par la zone 7 de la peau lorsqu'elle se déforme sous l'effet d'une dépression. La distance d entre le plan de la face d'extrémité 4 de la sonde (et donc la zone 7 avant déformation) et la face de la saillie 18 est avantageusement égale à 2.5 mm, ou voisine de cette valeur. La partie restante 21 du fond de la cavité 5 est située en retrait par rapport à la saillie 18 et forme une gorge annulaire. Un canal 21a, sensiblement parallèle à l'axe A est prévu dans la sonde et débouche, à une extrémité, dans la partie 21, et à son autre extrémité dans une chambre fermée 22 dont le volume est nettement supérieur à celui de la cavité 5. Une tubulure 23, de diamètre réduit par rapport à la tubulure 15, débouche dans la chambre 22 et est disposée sensiblement coaxialement à la tubulure 15. La tubulure 23 est reliée à un capteur de pression relative 24 permettant de déterminer la pression dans la chambre 22 et donc dans la cavité 5. Le capteur 24 est situé dans un boîtier de commande. La tubulure 23 traverse la paroi limitant la chambre 22 de manière étanche.

La chambre 22 est fermée, à sa partie supérieure, par un couvercle rapporté 25, fixé de manière étanche.

La réalisation des divers passages, canalisations et systèmes de conduits internes dans la sonde 3 peut nécessiter des perçages débouchant vers l'extérieur. Les ouvertures de ces perçages sur la surface externe de la sonde sont ensuite obturées par des bouchons tels que 26 réalisés en une matière appropriée.

Un clapet 27, schématiquement représenté, peut être prévu sur la tubulure 15 reliée à la pompe, et taré de manière à s'ouvrir lorsque l'orifice d'aspiration 2Ø est fermé par la peau 2 déformée.

Ceci étant, pour effectuer une mesure de l'élasticité de la peau, avec le dispositif de l'invention, on procède de la manière suivante. On délimite une zone 7 de la peau sur laquelle doit porter la mesure en plaçant la sonde 1 avec son axe A sensiblement perpendiculaire à la région considérée de la peau 2, le rebord 6 de la cavité 5 délimitant la susdite zone 7. On met alors en marche la pompe 17 qui crée une dépression, notamment dans les gorges 8 et 9, par aspiration d'air à travers les passages 11 et 12 ainsi que le système de conduit interne 13 de la sonde, et la tubulure 15.

La pompe 17 est choisie de manière à avoir un débit suffisant pour créer une dépression dans les gorges 8 et 9 qui applique de manière étanche la sonde 1 contre la peau 2. La dépression initiale nécessaire pour obtenir l'accrochage et l'étanchéité au niveau des gorges 8 et 9 est de l'ordre de 100 millibars.

Les rainures 8 et 9, maintenues sous dépression par les passages 12 et 11, assurent le maintien et l'étanchéité de la sonde sur la peau.

La pompe 17 a une capacité suffisante pour entretenir l'accrochage de la sonde pendant toute la mesure. Cette pompe 17 peut être constituée par une mini-pompe à palettes à grande vitesse (supérieure à 5ØØ tours/minute).

Lorsque la phase d'accrochage est réalisée, la peau est déformée au niveau des gorges 8 et 9 et est venue obturer l'extrémité des canaux 11 et 12.

La phase de mesure commence alors, l'aspiration se produisant essentiellement dans la cavité 5 à travers le passage 19.

La zone 7 de la peau 2 est aspirée rapidement; en se déformant, elle vient obturer l'orifice 2Ø. A ce moment, la pression dans la chambre 22, de grande capacité, reliée à la cavité 5, n'évolue plus dans le sens d'une diminution.

La valeur de cette pression mesurée à travers le tube 23, par l'intermédiaire du capteur 24, correspond à la dépression nécessaire pour déformer la zone 7 de peau d'une distance prédéterminée correspondant à la distance $\underline{d}$, dont la valeur est de 2,5 mm, dans l'exemple considéré.

Cette distance $\underline{d}$ et le diamètre D de la cavité 5 sont choisis avantageusement de manière que la dépression nécessaire à l'obturation de l'orifice 2Ø soit comprise entre 2Ø et 85 millibars.

Ainsi, selon l'invention, on mesure la dépression nécessaire pour créer une déformation constante, par exemple de 2,5 mm.

La sonde de mesure 3 est entièrement pneumatique et permet une mesure pratiquement instantanée.

Une petite pompe 17 à débit constant est suffisante.

La sonde 3 permet, avec la même pompe 17, de réaliser, automatiquement, deux fonctions à savoir, d'une part, l'étanchéité au niveau de la peau avec les rainures 8 et 9 et, d'autre part, la mesure à l'aide de la cavité 5.

## Revendications

1. Procédé pour mesurer l'élasticité d'une couche superficielle (2), en particulier de la peau, suivant lequel on délimite une zone (7) de cette couche, puis on crée, sur cette zone (7), une dépression pour provoquer une déformation de ladite zone (7), et on mesure la dépression nécessaire pour créer une déformation d'amplitude prédéterminée (d) de ladite couche (2), ladite déformation étant détectée par obturation d'un orifice (20) situé à une distance initiale correspondant à l'amplitude prédéterminée de déformation (d) de la couche superficielle (2), caractérisé par le fait que ledit orifice (20) est un orifice d'aspiration à travers lequel est crée la dépression et que la dépression mesurée est la dépression, qui existe dans un volume délimité par une gorge annulaire (21) et ladite zone (7) de la couche superficielle (2).

2. Dispositif pour mesurer l'élasticité d'une couche superficielle (2), en particulier de la peau, pour la mise en oeuvre du procédé selon la revendication 1, comprenant une sonde (3) munie d'une cavité (5) délimité par un rebord (6) propre à être appliqué sur la couche (2) à étudier pour définir la susdite zone (7), des moyens (17) pour créer une dépression dans la cavité (5) et provoquer la déformation de la zone (7), un orifice (20) prévu dans la susdite cavité (5), à une distance initiale correspondant à une amplitude prédéterminé de déformation (d) de la couche superficielle (2), cet orifice (20) étant disposé de manière à être obturé par la susdite couche lorsque la déformation atteint l'amplitude prédéterminée, et des moyens de mesure (24) de la dépression qui a produit cette déformation d'amplitude prédéterminée, caractérisé par le fait ledit orifice (20) étant un orifice d'aspiration à travers lequel est crée la dépression et que que la dépression mesurée est la dépression qui existe dans une gorge annulaire (21) dont est munie ladite cavité (5) et qui est en retrait par rapport audit orifice (20).

3. Dispositif selon la revendication 2, caractérisé par le fait que la cavité (5) comporte, dans son fond, une partie en saillie (18) traversée par un passage (19) de liaison avec un système de conduit interne (13) relié à une source de dépression (17), l'extrémité du passage (19) débouchant dans la cavité (5) constituant le susdit orifice (2Ø) destiné à être obturé par la zone déformée de la couche superficielle (2).

4. Dispositif selon l'une des revendications 2 ou 3, caractérisé par le fait que la zone centrale de la face d'appui (4) de la sonde contre la couche superficielle (2) est occupée par la cavité (5) et que cette face d'appui (4) comporte, autour de la cavité (5) des moyens (E) propres à réaliser une étanchéité entre la sonde (3) et la couche superficielle (2), lesdits moyens (E) comprenant au moins une gorge périphérique (8, 9) entourant la cavité (5) et reliée à une source de dépression (17), ladite source de dépression (17) étant la source destinée à créer une dépression dans la cavité (5).

5. Dispositif selon l'une des revendications 2 à 4, caractérisé par le fait qu'il comprend un clapet (27), monté sur une tubulure (15) reliée à une pompe (17), propre à s'ouvrir lorsque l'orifice d'aspiration (2Ø), prévu dans la cavité (5), est fermé par la couche superficielle (2) déformée.

6. Dispositif selon l'une des revendications 2 à 5, caractérisé par le fait que la sonde (3) comporte une chambre fermée (22), d'un volume supérieur à celui de la cavité (5), ladite chambre (22) étant reliée par un canal (21a) à ladite cavité (5).

7. Dispositif selon la revendication 6, caractérisé par le fait qu'il comporte une tubulure (23) débouchant dans la chambre (22) et reliée à un capteur de pression (24) pour permettre de mesurer la pression dans la chambre (22) et la cavité (5) lorsque cette dernière est fermée par la couche superficielle déformée.

8. Dispositif selon la revendication 6 ou 7, caractérisé par le fait que la sonde (3) a une forme cylindrique de révolution, dont la face transversale inférieure (4) est destinée à être appliquée contre la couche superficielle (2), la partie centrale de cette face inférieure (4) comportant la susdite cavité (5) entourée par un rebord (6) circulaire muni de deux gorges périphériques (8, 9) concentriques reliées à la source de dépression (17), la susdite chambre (22) étant prévue dans une zone intermédiaire, suivant la longueur du cylindre, cette chambre (22) étant reliée à la cavité (5) par un canal (21a) dans une région située à l'extérieur de la partie en saillie (18) du fond de la cavité (5), ladite chambre (22) étant en outre reliée, du côté opposé à la cavité (5), à une tubulure (23), coaxiale à une tubulure principale (15) de plus grand diamètre reliée à la source de dépression et communiquant à l'intérieur du cylindre avec un système de conduit interne (13) relié respectivement aux gorges périphériques (8, 9) et à l'orifice (2∅) débouchant dans la cavité (5).

**Patentansprüche**

1. Verfahren zur Bestimmung der Elastizität einer Oberflächenschicht (2), insbesondere der Haut, wobei man einen Abschnitt (7) der Schicht abgrenzt, auf diesem Abschnitt (7) einen Unterdruck erzeugt, um eine Deformation des Abschnittes (7) zu bewirken, und den erforderlichen Unterdruck für die Erzeugung einer Deformation mit einer vorbestimmten Amplitude (d) der Schicht (2) bestimmt, wobei die Deformation durch Verschließen einer Öffnung (20) detektiert wird, welche sich in einem anfänglichen, der vorbestimmten Amplitude (d) der Deformation. entsprechenden bestand von der Oberflächenschicht (2) befindet, **dadurch gekennzeichnet**, daß die Öffnung (20) eine Ansaugöffnung ist, durch die der Unterdruck erzeugt wird, und der gemessene Unterdruck derjenige Unterdruck ist, der in einem Volumen vorliegt, das durch eine ringförmige Auskehlung (21) und den Abschnitt (7) der Oberflächenschicht (2) begrenzt wird.

2. Vorrichtung zur Bestimmung der Elastizität einer Oberflächenschicht (2), insbesondere der Haut, zur Durchführung des Verfahrens gemäß Anspruch 1, mit einer Sonde (3) mit einer Ausnehmung (5), welche durch eine Randleiste (6) begrenzt wird, die man auf die zu untersuchende Schicht (2) aufsetzt, um den Abschnitt (7) zu definieren, Mitteln (17) um einen Unterdruck in der Ausnehmung (5) zu erzeugen und um die Deformation des Abschnitts (7) zu bewirken, einer Öffnung (20) in der Ausnehmung (5), die sich in einem anfänglichen,einer vorbestimmten Amplitude (d) der Deformation entsprechenden Abstand zur Oberflächenschicht (2) befindet, wobei die Öffnung (20) so angeordnet ist, daß sie von der Schicht verschlossen wird, wenn die Deformation die vorbestimmte Amplitude erreicht, und Mitteln (24) zur Messung des Unterdrucks, welcher diese Deformation mit der vorbestimmten Amplitude bewirkt, **dadurch gekennzeichnet**, daß die Öffnung (20) eine Ansaugöffnung ist, durch die der Unterdruck erzeugt wird, und der gemessene Unterdruck derjenige Unterdruck ist, der in einer ringförmigen Auskehlung (21) herrscht, die in der Ausnehmung (5) ausgebildet ist und die relativ zur Öffnung (20) zurückgesetzt ist.

3. Vorrichtung nach Anspruch 2, dadurch gekennzeichnet, daß die Ausnehmung (5) in ihrem Boden einen Vorsprung (18) umfaßt, welcher von einem Durchlaß (19) für ein innen angeordnetes Leitungssystem (13) durchquert wird, das mit einer Unterdruckquelle (17) verbunden ist, wobei das Ende des Durchlasses (19), welches in die Ausnehmung (5) einmündet, die Öffnung (20) bildet, welche von dem verformten Abschnitt der Oberflächenschicht (2) verdeckt wird.

4. Vorrichtung nach einem der Ansprüche 2 oder 3, dadurch gekennzeichnet, daß der zentrale beschnitt der Fläche (4) der Sonde zum Abstützen gegen die Oberflächenschicht (2) von der Ausnehmung (5) eingenommen wird, und daß die Abstützfläche (4) um die Ausnehmung (5) herum geeignete Mittel (E) aufweist, um eine Abdichtung zwischen Sonde (3) und Oberflächenschicht (2) auszubilden, wobei die Mittel (E) mindestens eine die Ausnehmung (5) umfassende umlaufende Auskehlung (8, 9) umfassen, die mit der Unterdruckquelle (17) verbunden ist, wobei die Unterdruckquelle (17) zur Erzeugung eines Unterdruckes in der Ausnehmung (5) dient.

5. Vorrichtung nach einem der Ansprüche 2 bis 4, dadurch gekennzeichnet, daß sie eine Verschlußklappe (27) umfaßt, welche an einem mit einer Pumpe (17) verbundenen Schlauch (15) angebracht ist, und sich öffnet, wenn die in der Ausnehmung (5) vorgesehene Ansaugöffnung (20) durch die verformte Oberflächenschicht (2) verschlossen wird.

6. Vorrichtung nach einem der Ansprüche 2 bis 5, dadurch gekennzeichnet, daß die Sonde (3) eine geschlossene Kammer (22) umfaßt, deren Volümen größer ist als,das der Ausnehmung (5), wobei die Kammer (22) über einen Kanal (21a) mit der Ausnehmung (5) verbunden ist.

7. Vorrichtung nach Anspruch 6, dadurch gekennzeichnet, daß sie einen Schlauch (23) umfaßt, der in die Kammer (22) mündet und mit einem Druckfühler (24) verbunden ist, um den Druck in der Kammer (22) und der Ausnehmung (5) zu bestimmen, wenn letztere durch die verformte Oberflächenschicht verschlossen ist.

8. Vorrichtung nach Anspruch 6 oder 7, dadurch gekennzeichnet, daß die Sonde (3) rotationszylindrisch ausgebildet ist und ihre untere Querfläche (4) zur Anwendung auf der Oberflächenschicht (2) bestimmt ist, wobei der zentrale Abschnitt dieser unteren Fläche (4) die Ausnehmung (5) umfaßt, welche durch eine kreisförmige Randleiste (6) umgrenzt wird, in welcher zwei mit der Unterdruckquelle (17) verbundene, umlaufende Auskehlungen (8, 9) konzentrisch ausgebildet sind, die Kammer (22) in einem zwischenliegenden beschnitt in Längsrichtung des Zylinders angeordnet ist, wobei die Kammer (22) mit der Ausnehmung (5) über einen Kanal (21a) in einem Bereich verbunden ist, der sich auf der Außenseite des Vorsprungs (18) im Boden der Ausnehmung (5) befindet, wobei die Kammer (22) auf der der Ausnehmung (5) gegenüberliegenden Seite mit einem Schlauch (23) verbunden ist, welcher koaxial zu einem Hauptschlauch (15) mit größerem Durchmesser angeordnet ist, der mit der Unterdruckquelle verbunden ist und mit dem Innenraum des Zylinders über ein inneres Leitungssystem (13) in Verbindung steht, welches wiederum mit den umlaufenden Auskehlungen (8, 9) bzw. mit der in die Ausnehmung (5) mündenden Öffnung (20) verbunden ist.

## Claims

1. A method for measuring the elasticity of a superficial layer (2), in particular of the skin, according to which a zone (7) of this layer is defined, then on this zone (7), a negative pressure is created to bring about a deformation of the said zone (7) and the negative pressure necessary to create a deformation of predetermined amplitude (d) of the said layer (2) is measured, the said deformation being detected by the obturating of an opening (20) located at an initial distance corresponding to the predetermined amplitude of the deformation (d) of the superficial layer (2), characterized in that the said opening (20) is a suction opening through which the negative pressure is created and that the measured negative pressure is the negative pressure which exists in a volume defined by an annular groove (21) and the said zone (7) of the superficial layer (2).

2. A device for measuring the elasticity of a superficial layer (2), in particular of the skin, for performing the method according to claim 1, including a sensor (3) provided with a cavity (5) defined by a rim (6), capable of being applied against the layer (2) to be studied to define the aforesaid zone (7), means (17) for creating a negative pressure in the cavity (5) and causing the deformation of the zone (7), an opening (20) provided in the aforesaid cavity (5) at an initial distance (d) corresponding to a predetermined amplitude of deformation of the superficial layer (2), this opening (20) being disposed in such a way as to be obturated by the aforesaid layer when the deformation attains the predetermined amplitude, and means (24) for measuring the negative pressure which has produced this deformation of a predetermined amplitude, characterized in that the said opening (20) is a suction opening through which the negative pressure is created and that the measured negative pressure is the negative pressure which exists in an annular groove (21) with which the said cavity (5) is provided, and which is indented with respect to the said opening (20).

3. A device according to claim 2, characterized in that the cavity (5) includes in its bottom, a projecting portion (18) penetrated by a passage (19) for communication with an internal conduit system (13) connected to a source of negative pressure (17), the end of the passage (19) discharging into the cavity (5) constituting the aforesaid opening (20) intended to be obturated by the deformed zone of the superficial layer (2).

4. A device according to one of claims 2 or 3, characterized in that the central zone of the face (4) on which the sensor rests against the superficial layer (2) is occupied by the cavity (5) and that this resting face (4) includes, around the cavity (5), means (E) capable of effecting a seal between the sensor (3) and the superficial layer (2), the said means (E) including at least one peripheral groove (8, 9) surrounding the cavity (5) and connected to a source of negative pressure (17), the said source of negative pressure (17) being the source intended to create a negative pressure in the cavity (5).

5. A device according to one of claims 2 to 4, characterized in that it includes a valve (27) mounted on a tube (15) connected to a pump (17), the valve being capable of being opened when the suction opening (20) provided in the cavity (5) is closed by the deformed superficial layer (2).

6. A device according to one of claims 2 to 5, characterized in that the sensor (3) includes a closed chamber (22) having a volume greater than that of the cavity (5), the said chamber (22) being connected via a conduit (21a) to the said cavity (5).

7. A device according to claim 6, characterized in that it includes a tube (23) discharging into the chamber (22) and connected to a pressure pickup (24) to permit the pressure in the chamber (22) and the cavity (5) to be measured when the latter is closed by the deformed superficial layer.

8. A device according to claim 6 or 7, characterized in that the sensor (3) has the shape of a cylinder of revolution, the lower transverse face (4) of which is intended to be applied against the superficial layer (2), the central portion of this lower face (4) including the aforesaid cavity (5) surrounded by a circular rim (6) provided with two concentric peripheral grooves (8, 9) connected to the source of negative pressure (17), the aforesaid chamber (22) being provided in an intermediate zone along the length of the cylinder, this chamber (22) being connected to the cavity (5) via a conduit (21a) in a region located outside the portion (18) projecting from the bottom of the cavity (5), the said chamber (22) being, moreover, connected, on the opposite side to the cavity (5), to the tube (23) which is coaxial with a primary tube (15) of larger diameter connected to the source of negative pressure and communicating on the inside of the cylinder with an internal conduit system (13) connected respectively to the peripheral grooves (8, 9) and to the opening (20) discharging into the cavity (5).

FIG. UNIQUE